# EUROPÄISCHE PATENTANMELDUNG

(11) **EP 4 234 531 A1**
(43) Veröffentlichungstag der Anmeldung: **30.08.2023**
(21) Anmeldenummer: 22158781.9
(22) Anmeldetag: 25.02.2022
(51) Int. Cl.: C07C 51/47, C07C 51/25, C07C 57/04, C07C 45/35, C07C 47/22

(54) **ABTRENNUNG VON ACRYLSÄURE MIT HILFE VON MEMBRANKONTAKTOREN**

(71) Anmelder: Evonik Superabsorber GmbH, 45128 Essen (DE)
(72) Erfinder: LIESTENER, Jessica, 45770 Marl (DE); HILLER, Christoph, 48249 Dülmen (DE); KUPPINGER, Franz-Felix, 45768 Marl (DE); SOBOLL, Sebastian, 45663 Recklinghausen (DE); KREIS, Peter, 44227 Dortmund (DE)
(74) Vertreter: Evonik Patent Association

(57) **Zusammenfassung**

Die Erfindung befasst sich mit der Extraktion von Acrylsäure aus wässrigen Strömen. Ihr liegt die Aufgabe zu Grunde ein Verfahren zur Extraktion von Acrylsäure aus wässrigen Strömen anzugeben, welches mit einem geringen Energieaufwand auskommt und welches längere Betriebszeiten erlaubt. Gelöst wird diese Aufgabe dadurch, dass zur Extraktion der Acrylsäure der wässrige Strom enthaltend die Acrylsäure mit einer Membran in Kontakt gebracht wird, deren von dem wässrigen Strom abgewandten Seite mit einem organischen Lösemittel beaufschlagt wird.

## Beschreibung

Acrylsäure ist eine Grundchemikalie, die industriell im großen Maßstab hergestellt wird. Sie dient unter anderem als Monomer für die Herstellung von Acrylaten.

Einen Überblick über die Herstellung von Acrylsäure und den daraus abgeleiteten Estern bieten: Ohara, T., Sato, T., Shimizu, N., Prescher, G., Schwind, H., Weiberg, O., Marten, K., Greim, H., Shaffer, T.D. and Nandi, P. (2021). Acrylic Acid and Derivatives. In Ullmann's Encyclopedia of Industrial Chemistry. https://doi.org/10.1002/14356007.a01_161.pub4.

Eine industriell bedeutsame Syntheseroute besteht darin, Propen in einem ersten Schritt zu Acrolein und sodann in einem zweiten Schritt zu Acrylsäure zu oxidieren. Die Oxidationsschritte können in getrennten Reaktoren an unterschiedlichen Katalysatoren oder integriert in einem Reaktor an einem Katalysator erfolgen. In der Regel erfolgt die Reaktion in der Gasphase. Das erhaltene Reaktionsgas enthält neben der gewünschten Acrylsäure auch viele weitere Nebenprodukte. Da die Nebenprodukte die Verwendbarkeit der Acrylsäure als Monomer für die Herstellung hochwertiger Acrylate sehr stark beeinträchtigen, ist es ein wesentliches technisches Entwicklungsziel der Acrylsäureproduktion, die Acrylsäure möglichst rein aus dem Reaktionsgas abzutrennen.

Meist wird dazu das Reaktionsgas mit einem flüssigen Absorber (in der Regel Wasser oder ein organisches Lösemittel) kontaktiert, sodass die Acrylsäure so zusammen mit einigen Nebenprodukten in einer Lösung vorliegt. Die Lösung wird dann mehrstufig destilliert, sodass konzentrierte Acrylsäure (crude acrylic acid - AA) erhalten wird. Diese wird sodann mit Kristallisationsprozessen weiter aufgereinigt, sodass am Ende hochreine Acrylsäure (glacial acrylic acid - GAA) vorliegt.

Dieser Prozess hat sich in jahrzehntelanger industrieller Praxis bewährt. Ein prinzipbedingter Nachteil besteht darin, dass Acrylsäure äußerst reaktionsfreudig ist und sogar zur Selbstpolymerisation neigt. Daher muss ein großer Aufwand betrieben werden, um die Reaktion der Acrylsäure mit sich selbst während ihrer Aufarbeitung zu vermeiden. Dies geschieht unter anderem durch Zugabe von Inhibitoren, welche die Reaktion unterdrücken. Dies gelingt aber nicht immer vollständig, sodass sich über einen längeren Betriebszeitraum Polyacrylate in den Destillationskolonnen ablagen. Um die Betriebssicherheit aufrecht zu erhalten, muss die Destillation der Acrylsäure regelmäßig unterbrochen, die Kolonnen geleert und manuell von Polyacrylaten gereinigt werden. Dies ist ein sehr aufwändiger Vorgang, der die Kosten für die Aufarbeitung der Acrylsäure nennenswert erhöht. Darüber hinaus ist für die Destillation von Acrylsäure auch viel Heizenergie notwendig, welche die Produktionskosten steigern und den CO₂-Fußabdruck der Acrylsäure und ihrer Folgeprodukte vergrößern.

Es besteht daher ein großes Interesse daran, die Aufreinigung von Acrylsäure weniger aufwändig und gleichzeitig energieeffizienter zu ermöglichen.

Eine Möglichkeit, den Einsatz von Heizenergie zu verringern, besteht in dem Einsatz von Extraktionsprozessen. In Extraktionsprozessen wird ein wässriger Absorber enthaltend die Acrylsäure mit einem organischen Extraktionsmedium kontaktiert. Die Acrylsäure wird dadurch in dem organischen Lösemittel angereichert. Sodann ist noch eine Abtrennung der organischen Phase von der wässrigen Phase erforderlich und schließlich die Destillation der organischen Phase, um die Acrylsäure zu erhalten. Ein Vorteil des beschriebenen Extraktionsprozesses gegenüber einer reinen Destillation besteht darin, dass weniger Heizenergie erforderlich ist. Dies ist dadurch begründet,dass das Lösemittel in der Regel eine niederigere Vedampfungsenthalpie aufweist als Wasser. Die destillative Trennung der Acrylsäure von dem Lösemittel ist daher energetisch günstiger als die Abtrennung von Acrylsäure aus Wasser.

Ein Verfahren zur Extraktion von Acrylsäure mit organischen Lösemitteln ist in der EP1466885A2 beschrieben. Als Extraktionsmedium wird ein Gemisch aus Isopropylacetat und Toluol eingesetzt, als Kontaktor wird eine KARR-Kolonne erwähnt. In KARR-Kolonnen werden die organische und die wässrige Phase von rotierenden Aktuatoren dispergiert, um eine große Kontaktfläche an den Phasengrenzen zu erreichen. Prinzipbedingter Nachteil der KARR-Kolonnen ist wiederum der Bedarf an mechanischer Energie, die erforderlich ist, um die Aktuatoren zu drehen. Außerdem sind die beweglichen Teile der KARR-Kolonne besonders anfällig gegen Polymerisate: Deswegen haben KARR-Kolonnen in der Acrylsäure vergleichsweise engmaschige Abschaltzeiten. Darüber hinaus erfordert eine KARR-Kolonne weiterhin eine Phasentrennung nach erfolgter Extraktion.

Als eine weitere Möglichkeit zur Abtrennung von Acrylsäure aus wässrigen Strömen kommt die Membranseparation in Betracht. So beschreibt die US5635071A die Abscheidung von Carbonsäuren aus wässrigen Strömen mit Hilfe einer Nanofiltrationsmembran. Das Verfahren ist druckgetrieben, vorzugsweise bei 300 psig Transmembrandruck und wird bei einer Temperatur von 40 bis 50 °C durchgeführt. Konkret wird ein Gemisch aus Essigsäure und Ameisensäure durch die Membran abgeschieden. Das Membranmaterial wird nicht genau angegeben; es wird lediglich auf die Verwendbarkeit einer Entsalzungsmembran von Desalination Systems Inc. of California, erhältlich unter der Marke DS-5-DK8040 hingewiesen. Was das für ein Membranmaterial konkret ist, wird nicht gesagt. Die Auswahl des Membranmaterials ist für eine Fachperson auf dem Gebiet der Membrantechnologie von entscheidendem Interesse. Insoweit ist der Offenbarungsgehalt der US5635071A unvollständig. Darüber hinaus ist auch nicht klar, ob sich die Entsalzungsmembran auch für die Abscheidung von Acrylsäure eignet, weil dies in der Schrift nicht untersucht wurde. Schließlich ist die Temperatur, bei dem die Nanofiltration durchgeführt wird, noch recht hoch, sodass mit einer gesteigerten Polymerisationsneigung zu rechnen ist. Dieser Effekt dürfte noch verstärkt werden, wenn die Nanofiltrationsmembran undurchlässig für den Inhibitor wäre. In dem Fall ist permeatseitig gewiss mit einer Autopolymerisation der Acrylsäure zu rechnen. Im Ergebnis ist es zweifelhaft, ob die in US5635071A offenbarte technische Lehre ausreichend ist, eine membrangestützte Separation von Acrylsäure aus wässrigen Lösungen zu realisieren.

In Hinblick auf diesen Stand der Technik liegt der Erfindung die Aufgabe zu Grunde, ein Verfahren zur Extraktion von Acrylsäure aus wässrigen Strömen anzugeben, welches mit einem geringen Energieaufwand auskommt und welches längere Betriebszeiten erlaubt.

Gelöst wird diese Aufgabe dadurch, dass der wässrige Strom enthaltend die Acrylsäure mit einer Membran in Kontakt gebracht wird, deren von dem wässrigen Strom abgewandten Seite mit einem organischen Lösemittel beaufschlagt wird.

Ein solches Verfahren ist ein erster Gegenstand der Erfindung.

Die Erfindung basiert auf der Grundidee, dass sich zur Extraktion der Acrylsäure ein so genannter Membrankontaktor einsetzen lässt. Ein Membrankontaktor ist ein Apparat, der von einer porösen Membran in zwei Kompartimente geteilt wird: Die so genannte Shell und das sogenannte Lumen. Die Shell liegt diesseits der Membran, währenddessen das Lumen jenseits der Membran liegt. Die Membran weist materialbedingt eine unterschiedliche Benetzbarkeit für organische und wässrige Medien auf. Wenn die beiden Kompartimente jeweils mit dem wässrigen bzw. dem organischen Medium beaufschlagt werden, füllen sich die Poren der Membran mit der Phase, für die das Membranmaterial eine höhere Benetzbarkeit aufweist. Aufgrund der Oberflächenspannung zwischen den beiden Medien an der Grenzfläche kommt es zu keiner Vermischung: Die Überflächenspannung der beiden Medien zueinander verhindert ein Durchbrechen. Die für die Extraktion erforderliche Kontaktfläche zwischen den beiden Phasen wird über die Poren der Membran definiert. Im Ergebnis ermöglicht der Membrankontaktor eine flüssig/flüssig Extraktion aus der wässrigen Phase in die organische Phase an einer definierten Kontaktfläche ohne Vermischung der Phasen.

Der Vorteil des Membrankontaktors besteht folglich darin, dass eine Extraktion durchgeführt werden kann, ohne eine anschließende Phasentrennung vornehmen zu müssen.

Geeignete Membrankontaktoren sind unter anderem aus der WO 2008088293 A1 oder auch aus der EP 3444021 B1 bekannt und zudem kommerziell erhältlich.

Vorzugsweise wird eine Membran verwendet, die poröses Polytetrafluorethylen (PTFE) enthält oder daraus besteht. Das PTFE kann hydrophiliert sein, muss es aber nicht.. Normalerweise ist PTFE hydrophob. Es ist im Groben nicht wichtig, ob eine hydrophilierte oder nicht hydrophilierte PTFE-Membran verwendet wird. Wichtig bei der Auswahl des Membranmaterials ist nur, dass von zwei Phasen, die an der Membran in Kontakt gebracht werden sollen, nur eine die Oberfläche der Membran benetzt. Wenn dann auch die beiden Medien untereinander eine Oberflächenspannung besitzen -was bei einem organischen Medium einerseits und einem wässrigen Medium andererseits der Fall ist - verhindert das den Durchbruch durch die Membran. Mit Durchbruch ist hier gemeint, dass die organische Phase durch die Poren in die wässrige Phase strömt. Dies wird durch den höheren Druck im Wasser und die Oberflächenspannung verhindert.

Darüber hinaus ist es grundsätzlich vorteilhaft, ein Membranmaterial auszuwählen, das benetzbar ist mit dem Medium, in welchem der Diffusionskoeffizient der auzutauschenden Komponente höher ist.

Wichtig ist jedoch die Porosität des Membranmaterials. Vorzugsweise weist das trennaktive Material eine Porosität von 25 % bis 75 % auf. Vorzugsweise liegt die Porosität bei etwa 50 %. Die Poren sollten einen Pordendurchmesser zwischen 0,2 µm und 0.4 µm aufweisen. Vorzugsweise besteht die Membran komplett aus entsprechend porösem PTFE.

Vorzugsweise liegt die Membran als ein Hohlfaser-Modul vor. Hohlfaser-Module umfassen ein Bündel einer Vielzahl von parallel verschalteten Hohlfaser-Membranen. Eine Hohlfaser-Membran hat die Gestalt einer Rohrleitung, auf deren Lumenseite (innen) das eine Medium fließt, währenddessen auf der Außenseite (Shell) das andere Medium fließt. Die Shellseite aller Hohlfasern bilden einen gemeinsamen Raum, wohingegen jede Hohlfaser ein individuelles Lumen aufweist. Vorzugsweise befindet sich lumenseitig der wässrige Strom währenddessen auf der Shellseite das organische Lösemittel fließt. Es ist auch umgekehrt möglich. Vorzugsweise fließen organisches Lösemittel und wässriger Strom gegenläufig durch das Hohlfaser-Modul (Gegenstrombetrieb). Dadurch wird das Konzentrationsgefälle besser aufrechterhalten.

Ein Vorteil des Extraktionsverfahrens ist, dass es sich bei Umgebungstemperatur durchführen lässt. Dadurch wird im Vergleich zu einer Destillation Heizenergie eingespart. Vorzugsweise wird die Extraktion bei einer Temperatur von 20°C bis 30°C durchgeführt, weil dann in der Regel keine Heizenergie erforderlich ist. Außerdem ist die Polymerisationsneigung der Acrylsäure bei solch niedrigen Temperaturen geringer ausgeprägt, was die Betriebssicherheit erhöht, die Abschaltintervalle verlängert und den Inhibitorbedarf reduziert. Alternativ kann das Verfahren auch bei einer Temperatur von 20°C bis 50°C durchgeführt werden.

Als organisches Lösemittel wird eine Substanz verwendet, die einerseits gut Acrylsäure absorbiert, andererseits die Membran nicht angreift. Als organisches Lösemittel eignen sich insbesondere Toluol, n-Heptan, Essigsäureisobutylester, Essigsäure-n-propylester, Essigsäureisopropylester, 2-Pentanon, Methylisobutylketon. Es können auch Mischungen dieser Substanzen als Lösemittel eingesetzt werden.

Vorzugsweise wird Toluol als organisches Lösemittel eingesetzt. Toluol erfüllt die Anforderungen sehr gut und ist als Massenchemikalie leicht verfügbar. Eine Mischung mit weiteren Substanzen ist dann nicht erforderlich. Mithin besteht das frische organische Lösemittel vorzugsweise aus 95 Gew.-% bis 100 Gew.-% Toluol. Der Rest können Verunreinigungen sein.

Es sei klargestellt, dass die im vorliegenden Verfahren eingesetzte Membran unbewegt ist. Dies bedeutet, es wird keine mechanische Leistung benötigt, um die Membran gegenüber der Umgebung zu bewegen. Insoweit besteht ein Vorteil gegenüber einer KARR-Kolonne, die bewegliche Teile umfasst. Darüber hinaus ist eine unbewegte Membran weniger anfällig gegen Blockaden durch ungewollte Polymerisation. Die für die Durchströmung der Membran mit dem wässrigen Medium erforderliche mechanische Leistung ist vernachlässigbar, weil der wässrige Strom einem kontinuierlichen Prozess ohnehin gepumpt werden muss.

Gemäß einer bevorzugten Weiterbildung des Verfahrens wird das organische Lösemittel im Kreislauf zirkuliert, wobei der Kreislauf die Beaufschlagung der Membran mit dem organischen Lösemittel und eine destillative Abtrennung des organischen Lösemittels aus der Acrylsäure aus umfasst. Die geschlossene Kreislaufführung des Toluols lässt sich besonders gut in einen kontinuierlichen Prozess zur Acrylsäure- oder Acroleinproduktion einbinden. Das Toluol geht dabei aus dem System nicht verloren, wodurch der Prozess besonders nachhaltig ist. Dieser Prozess ist besonders geeignet dafür, den Wertstoff Acrylsäure von Toluol zu reinigen.

Es gibt verschiedene Entscheidungskriterien, welches Medium lumenseitig und welches shellseitig geführt wird. Man kann dafür die Grenzfläche betrachten. Die Hohlfasern haben außen einen höheren Durchmesser als innen. Liegt also die Grenzfläche außen (benetzendes Medium wird Lumenseitig gefahren), so ist die Grenzfläche etwas höher als umgekehrt. Ein anderer Aspekt ist die strömungsdynamische Betrachtung. Im Lumen ist die Strömung gradliniger als auf der Shell. Aus diesem Grund macht es Sinn, den zu extrahierenden Strom durch das Lumen zu führen, um Rückvermischung durch Verwirbelungen in der Strömung mit weniger abgereichertem Produkt vor dem Austritt aus dem Kontaktor zu verhindern. Im Lichte dieser Überlegungen wird das organische Lösemittel vorzugsweise shellseitig gefahren, währenddessen der wässrige Strom lumenseitig gefahren wird.

Sofern eine hydrophobe-Membran eingesetzt wird, ist es notwendig, den wässrigen Strom mit einem höheren Druck durch das Membranmodul zu fahren als das organische Lösemittel. Konkret ist es bevorzugt, den Prozess mit einem Druckgefälle zu fahren, welches zwischen der wässrigen Seite und der organischen Seite der Membran herrscht, wobei der Druck auf der wässrigen Seite zwischen 1000 Pa und 10000 Pa höher liegt als der Druck auf der organischen Seite.

Ein solch leichter Überdruck auf der Seite des nicht benetzenden Mediums verhindert ein Durchbrechen der benetzenden Flüssigkeit. Bei einer nicht hydrophilierten Membran sollte also immer auf der Seite der wässrigen Phase ein höherer Druck herrschen als auf der Seite der benetzenden (organischen) Phase. Beim Anfahren des Prozesses muss dann auf der Seite der wassrigen Phase begonnen werden. Würde man mit der benetzenden Phase starten, so würde diese durch die Poren auf die andere Seite der Memrban fließen, weil es keinen Gegendruck gibt. Dementsprechend ist es egal ob der höhere Druck shellseitig oder lumenseitig ist. Wichtig ist nur, dass auf der Seite der nicht benetztenden Flüssigkeit der Druck höher ist als auf der benetztenden Seite. Im Übrigen ist das Druckgefälle nicht die treibende Kraft. Die treibende Kraft ist der Konzentrationsgradient.

Eine bevorzugte Weiterbildung des erfindungegemäßen Verfahrens sieht daher ein Druckgefälle vor, welches zwischen den beiden Seiten der vorliegend nicht hydrophilierten Membran herrscht, wobei der Druck auf der dem wässrigen Strom abgewandten (organischen) Seite zwischen 1000 Pa und 10000 Pa niedriger liegt als der Druck auf der dem wässrigen Strom zugewandten (wässrigen) Seite.

Sofern eine hydrophilierte Membran eingesetzt wird, ist das Druckgefälle entsprechend umzukehren.

Eine alternative Weiterbildung des erfindungegemäßen Verfahrens sieht daher ein Druckgefälle vor, welches zwischen den beiden Seiten einer hydrophilierten Membran herrscht, wobei der Druck auf der dem wässrigen Strom abgewandten (organischen) Seite zwischen 1000 Pa und 10000 Pa höher liegt als der Druck auf der dem wässrigen Strom zugewandten (wässrigen) Seite.

Um die unerwünschte Polymerisation von Acrylsäure während der Extraktion zu vermeiden, ist es angezeigt, die Extraktion in Gegenwart eines Inhibitors durchzuführen. Der Inhibitor muss auf beiden Seiten der Membran anwesend sein. Aufgrund der wässrigen Umgebung auf der einen Seite und der organischen Umgebung auf der anderen, ist es möglich, unterschiedliche Inhibitoren zu verwenden, die entsprechend stabiler in wässriger bzw. organischer Umgebung sind. Dementsprechend sieht eine Weiterbildung des Verfahrens es vor, dass der wässrige Strom einen ersten Inhibitor enthält und dass das organische Lösemittel einen zweiten Inhibitor enthält, wobei der erste Inhibitor und der zweite Inhibitor gleich oder unterschiedlich sind.

Als erster Inhibitor im wässrigen Strom kommen insbesondre Hydrochinon (1,4-Dihydroxybenzol) und/oder Hydrochinonmonomethylether (MEHQ) in Betracht, als zweiter Inhibitor in dem organischen Lösemittel kann 4-Hydroxy-2,2,6,6-tetramethylpiperidinyloxyl (4-HT) verwendet werden.

Das hier beschriebene Extraktionsverfahren kann auch mit klassischen Extraktionsverfahren inklusive Phasentrennung kombiniert werden, etwa dann, wenn Acrylsäure in Mengen abzutrennen ist, für welche die Leistungsfähigkeit der Membrankontaktoren nicht ausreicht. Dann würde in einem ersten Schritt klassisch mit Phasentrennung extrahiert und dann in einem zweiten Schritt erfindungsgemäß mit Membrankontaktor und ohne Phasentrennung. Der zweite erfindungsgemäße Extraktionsschritt würde dann eine Feinreinigung darstellen. Letztendlich ist die Wahl des passenden Setups eine Frage der Wirtschaftlichkeit.

Das hier beschriebene Extraktionsverfahren funktioniert im Übrigen auch in die entgegensetzte Richtung, nämlich um Acrylsäure aus einem organischen Strom in einen wässrigen Strom zu extrahieren. Erstaunlicherweise funktioniert der umgekehrte Weg sogar ohne die Benetzbarkeit der Membran umgekehrt einzustellen. Es kommt scheinbar nicht darauf an, auf welcher Seite die Membran welche Benetzbarkeit aufweist; entscheidend ist, dass auf einer Seite die Benetzbarkeit der Membran für Wasser größer ist als für das organische Lösemittel.

Ein zweiter Gegenstand der Erfindung ist mithin Verfahren zur Extraktion von Acrylsäure aus organischen Strömen, dadurch gekennzeichnet, dass ein organischer Strom enthaltend Acrylsäure mit einer Membran in Kontakt gebracht wird, deren von dem organischen Strom abgewandten Seite mit einem wässrigen Lösemittel beaufschlagt wird.

Vorzugsweise wird das erfindungsgemäße Extraktionsverfahren in der industriellen Produktion von Acrylsäure eingesetzt. Ein weiterer Gegenstand der Erfindung ist mithin ein Verfahren zur Herstellung von Acrylsäure, umfassend einen ersten Reaktionsschritt, bei dem Propen mit Sauerstoff zu Acrolein umgesetzt wird, einen zweiten Reaktionsschritt, bei dem Acrolein mit Sauerstoff zu Acrylsäure umgesetzt wird, einen Absorbtionsschritt, bei dem Acrylsäure in einen wässrigen Strom in absorbiert wird und einen Extraktionsschritt, bei dem die Acrylsäure aus dem wässrigen Strom in hier beschriebener Weise in ein organisches Lösemittel extrahiert wird.

Konkret kann der Membrankontaktor anstatt einer KARR-Kolonne verwendet werden. Alternativ kann eine Kombination aus einer Extraktionskolonne ohne Energieeintrag mit einem Membrankontaktor in Reihe zu eingesetzt werden. Außerdem kann der Membrankontaktor in den Abwasserströmen der Acrylsäureproduktion verwendet werden, um restliche Acrylsäure mit Hilfe eines organischen Lösemittels zurück zu gewinnen.

Ebenso kann das erfindungsgemäße Extraktionsverfahren in der industriellen Produktion von Acrolein eingesetzt werden. Ein weiterer Gegenstand der Erfindung ist mithin ein Verfahren zur Herstellung von Acrolein, umfassend einen Reaktionsschritt, bei dem Propen mit Sauerstoff zu Acrolein und Acrylsäure umgesetzt wird und einen Absorbtionsschritt, bei dem Acrylsäure in einen wässrigen Strom absorbiert wird und einen Extraktionsschritt, bei dem die Acrylsäure aus dem wässrigen Strom in hier beschriebener Weise in ein organisches Lösemittel extrahiert wird.

Der Membrankontaktor kann insbesondere in den Abwasserströmen der Acroleinproduktion verwendet werden, um restliche Acrylsäure mit Hilfe eines Lösemittels zurück zu gewinnen.

In analoger Weise lässt sich das erfindungsgemäße Verfahren bei der Herstellung von Acrylaten, insbesondere von Butylacryltat einsetzen, weil bei solchen Verfahren auch wässrige Ströme enthaltend Acrylsäure anfallen. Die Acrylsäure kann mit dem erfindungsgemäßen Extraktionsverfahren zurückgewonnen werden.

Die Erfindung soll nun anhand eines Ausführungsbeispiels näher erläutert werden. Hierfür zeigen:
- Figur 1:: Vereinfachtes Fließbild des Versuchsaufbaus;
- Figur 2:: Beladungsverlauf der Acrylsäure in wässriger und organischer Phase;
- Figur 3:: Höhe der Sedimentationslinie gegen Zeit nach Dispersion in den Schüttelversuchen (nicht erfindungsgemäß).

In dem hier betrachteten Anwendungsfall soll Acrylsäure aus einem wässrigen Strom extrahiert werden.

Beispielhaft betrachtet wurde die Extraktion von Acrylsäure aus Wasser mit Hilfe von Toluol an einer PTFE-Membran. Konkret wurde ein Membrankontaktor in Gestalt eines Hohlfaser-Moduls, bezogen von Fa. Memo3 GmbH, CH-4313 Möhlin eingesetzt. Der Anwendungsfall in die andere Richtung (Extraktion von Acrylsäure aus einem PTFE-benetzenden Strom mit Hilfe von Wasser) ist auch denkbar. Auch ist es denkbar eine hydrophilierte PTFE-Membran zu verwenden. Die Extraktion von 60% -iger Acrylsäure in Wasser mit Toluol hat in den Versuchen funktioniert. In diesen Versuchen wurden bei Raumtemperatur die beiden Lösungen in Kreisfahrweise aus ihrer Vorlage immer wieder über den Kontaktor gefahren und anschließend wieder in der gleichen Vorlage gesammelt.

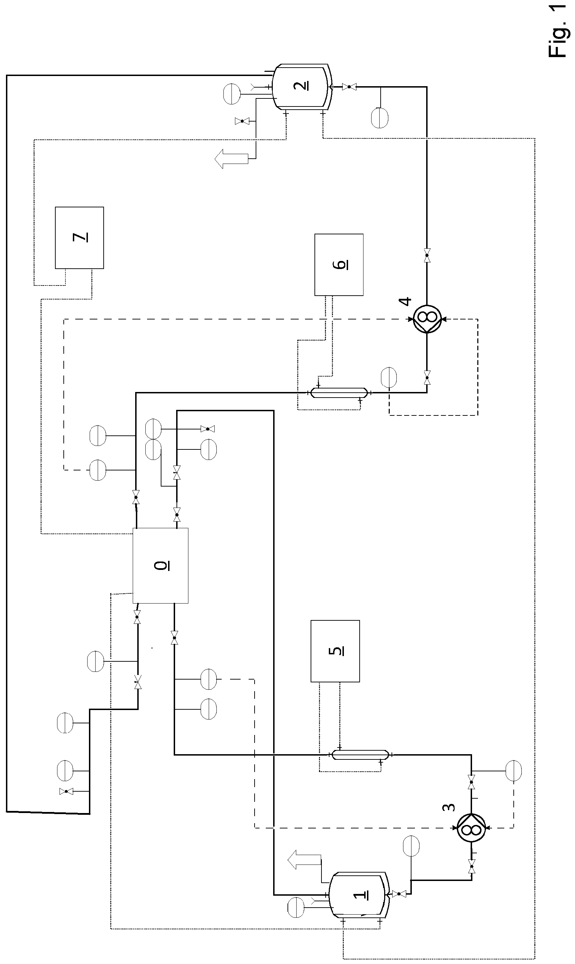

Ein vereinfachtes Verfahrensfließbild des Versuchsaufbaus ist in Figur 1 dargestellt.

Zentrales Element ist der untersuchte Membrankontaktor 0. Der Membrankontaktor 0 weist keine bewegten Bauteile auf. In einem ersten Behälter 1 ist Wasser mit dem jeweiligen Anteil an Acrylsäure vorgelegt. In einem zweiten Behälter 2 befindet sich als organisches Lösemittel Toluol. Mit einer ersten Pumpe 3 wird ein wässriger Strom enthaltend Acrylsäure aus dem ersten Behälter 1 in das Lumen des Membrankontaktors 0 gefördert. Mit einer zweiten Pumpe 4 wird das organische Lösemittel (Toluol) aus dem zweiten Behälter 2 in die Shell des Membrankontaktors 0 gefördert, sodass ein organischer Strom entsteht.

Mithilfe eines ersten Thermostaten 5 mit angeschlossenem Wärmetauscher wird die Temperatur des wässrigen Stroms in das Lumen des Membrankontaktors 0 eingestellt. Mithilfe eines zweiten Thermostaten 6 mit angeschlossenem Wärmetauscher wird die Temperatur des organischen Lösemittels in die Shell des Membrankontaktors 0 eingestellt. Ein dritter Thermostat 7 stellt die Temperatur des Membrankontaktors 7 und der beiden Behälter 1, 2 ein. Die Mess- und Regelleitungen der Temperaturregelung sind in Figur 1 gepunktet dargestellt.

Neben der Temperaturregelung mit Hilfe der drei Thermostaten 5, 6, 7 besitzt der Versuchsaufbau eine Strömungsregelung, welche die Drücke und Volumenströme des wässrigen Stroms und des organischen Stroms regelt. Dies erfolgt über die jeweiligen Pumpen 3, 4 und zahlreiche Ventile, die in Figur 1 eingezeichnet, aber nicht beziffert sind. Die zugehörigen Regler und Sensoren sind ebenfalls eingezeichnet, aber nicht einzeln beziffert. Die Mess- und Regelleitungen der Strömungsregelung sind in Figur 1 gestrichelt dargestellt.

Die Leitungen, durch die der wässrige bzw. organische Strom fließen, sind in Figur 1 durchgezogen dargestellt. Der wässrige Strom enthaltend Acrylsäure und das organische Lösemittel fließen im Gegenstrom durch den Membrankontaktor 0 und werden in ihren jeweiligen Behälter 1, 2 rezykliert.

Im Membrankontaktor 0 erfolgt die Extraktion der Acrylsäure aus dem wässrigen Strom in das organische Lösemittel. Die Acrylsäure wandert dabei von der Lumenseite auf die Shellseite der Membran. Acrylsäure wird im Wasser abgereichert und im Toluol angereichert.

Im ersten Behälter 1 wurde etwa 1 kg 60 %-ige Acrylsäure in Wasser, stabilisiert mit 500 ppm 4-Hydroxy-2,2,6,6- tetramethylpiperidinyloxyl (4-HT), vorgelegt und im Kreis über den Kontaktor 0 gefahren. Die wässrige Phase wird in dem als Hohlfasermodul ausgeführten Kontaktor lumenseitig gefahren. Shellseitig wird Toluol, ebenfalls stabilisiert mit 500 ppm 4-HT im Kreis gefahren. Das Toluol wird im zweiten Behälter 2 mit etwa 2L vorgelegt und im Kreis über den Kontaktor gefahren. Im Membrankontaktor 0 werden die wässrige Phase und die organische Phase im Gegenstrom miteinander kontaktiert. Der Versuch findet bei 25°C statt. Lumenseitig wird ein Druck von etwa 65 mbarü (6500 Pa über dem Druck auf der Shellseite) eingestellt, um zu verhindern, dass das Toluol durchbricht. Die beiden Behälter 1 und 2 wurden regelmäßig beprobt, um den Acrylsäureanteil in den beiden Lösungen zu bestimmen.

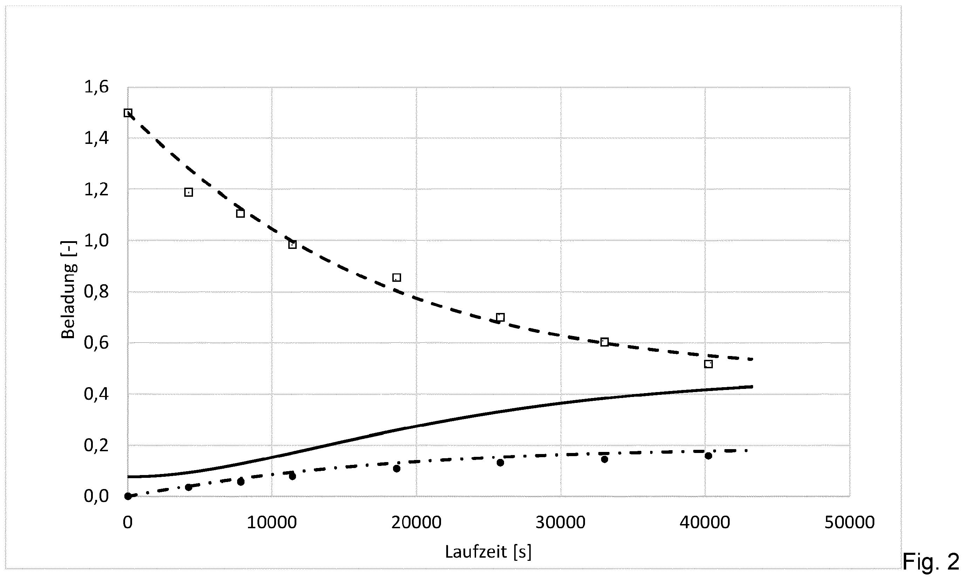

In dem in Figur 2 gezeigten Diagramm sind die experimentell ermittelten Beladungen der beiden Phasen dargestellt. In dem Diagramm ist die Massenbeladung über die Laufzeit des Versuches aufgezeichnet. Die quadratischen Punkte (_{□}) betreffen die wässrige Phase, die runden Punkte (•) betreffen die organische Phase. Rechnerisch interpoliert ist die Beladung für die wässrige Phase als gestrichelte Linie dargestellt. Rechnerisch interpoliert ist die Beladung für die organische Phase als strichpunktierte Linie dargestellt. Die berechnete Gleichgewichtslinie für die wässrige Phase ist als durchgezogene Linie dargestellt.

Der Versuch hat gezeigt, dass eine Abreicherung der Acrylsäure aus dem Wasser mit Toluol als Extraktionsmittel über den Membrankontaktor innerhalb von 11 h nahezu bis zum ersten Gleichgewichtspunkt stattgefunden hat. In den 11 h konnte kein Trennleistungseinbruch z.B. durch auftretende Polymerisation und ggf. damit verbundenem Einbruch der Porosität der Membran, beobachtet werden. Auch konnte optisch kein Quellen der Membran festgestellt werden. Es trat keine Polymerisation auf. Dies ist dadurch zu erklären, dass der Versuch bei Raumtemperatur stattfand und die Inhibitoren in den beiden Lösungen eine Polymerisation verhindern.

Die Machbarkeit der Extraktion von Acrylsäure aus Wasser mit Hilfe von Toluol über einen Membrankontaktor unter Inhibierung mittels 4-HT ist mit diesem Versuch gezeigt worden. Es konnte ein Stoffdurchgangskoeffizient k in Höhe von 2,4*10⁻⁶ m/s bestimmt werden, dessen theoretische Konzentrationsverläufe von Acrylsäure in Wasser und Toluol nur mit geringen Abweichungen mit den experimentellen Daten übereinstimmen. Die mit Hilfe des Stoffdurchgangskoeffizienten ermittelten theoretischen Kurven sind in Figur 2 in den unterbrochenen Linien dargestellt. In der durchgezogenen Linie ist für jeden Zeitpunkt die ermittelte Gleichgewichtsbeladung in der wässrigen Phase dargestellt.

In Tabellen 1a bis 1d sind die wichtigsten Versuchsdaten dargestellt. Tabelle 1a zeigt die im Versuch verwendeten Ansätze, Tabelle 1b zeigt die Durchflüsse in der Versuchsdurchführung, in Tabelle 1c sind die allgemeinen Verfahrensvorschriften niedergelegt. Die Versuchsergebnisse sind in Tabelle 1d zusammengetragen.

**Tabelle 1a: Ansätze in der Versuchsdurchführung**

| | Toluol | AA | Wasser | 4HT |
|---|---|---|---|---|
| wässrige Phase | | 60% | 40% | 500ppm |
| Organik | 100% | | | 500ppm |

**Tabelle 1b: Durchflüsse in der Versuchsdurchführung**

| | | | | | | Durchfluss |
|---|---|---|---|---|---|---|
| | | | | | | Soll |
| | | | | | | kg/h |
| Einwaage wässrige Phase: | | 998 | g | (Soll=1kg) | Lumen | 5 |
| Einwaage org. Phase | | *2135* | g | (Soll=2,1kg) | Shell | 5 |

**Tabelle 1c: Verfahrensvorschriften**

| | | | | | |
|---|---|---|---|---|---|
| Versuchstemperatur: | 25°C | | | | |
| Versuchsdauer: | 24h | | | | |
| Proben intervall | alle 2 h | | | | |
| Probe menge | 10mL für wässr. Phase | | | | |
| | 10mL für org. Phase | | | | |
| Bemerkung: | 1L Stopperlösung (Wasser mit 500ppm 4-HT) vorhalten | | | | |

**Tabelle 1d: Versuchsergebnisse**

| **Versuchszeit** | **PIR301** | **PIR302** | **PIR303** | **Differenzdruck** | **Stand** | **Stand** | **FIR 202** | **FIR 204** |
|---|---|---|---|---|---|---|---|---|
| | Lumen ein | Lumen aus | Shell ein | | Lumen | Shell | Dichte Lumen | Dichte Shell |
| hh:mm | mbar | mbar | mbar | | cm | cm | g/mL | g/mL |
| | | | | | | | | |
| | 1,070 | 1,01 | 1,024 | 0,046 | 5 | 15 | 1,043 | 0,861 |
| 1 | 1,070 | 1,01 | 1,018 | 0,052 | 5,3 | 14,6 | 1,042 | 0,862 |
| 2 | 1,055 | 1,01 | 1,016 | 0,039 | 6,2 | 14 | 1,041 | 0,863 |
| 3 | 1,064 | 1,01 | 1,016 | 0,048 | 7,2 | 13,6 | 1,039 | 0,864 |
| 4 | 1,061 | 1,01 | 1,018 | 0,043 | 7,9 | 13,5 | 1,039 | 0,868 |
| 5 | 1,060 | 1,01 | 1,018 | 0,042 | 8,4 | 13 | 1,039 | 0,870 |
| 6 | 1,064 | 1,01 | 1,019 | 0,045 | 9 | 12,8 | 1,039 | 0,872 |
| 7 | 1,064 | 1,01 | 1,019 | 0,045 | 9,3 | 12,5 | 1,038 | 0,873 |
| 8 | 1,060 | 1,01 | 1,019 | 0,041 | 10 | 12,5 | 1,037 | 0,874 |
| 9 | 1,062 | 1,01 | 1,0172 | 0,045 | 10,5 | 12,5 | 1,03544 | 0,875 |
| 10 | 1,062 | 1,01 | 1,0167 | 0,045 | 11 | 12,5 | 1,0344 | 0,876 |
| 11 | 1,060 | 1,01 | 1,0169 | 0,043 | 11,5 | 12,5 | 1,03336 | 0,877 |

Als Vergleich wurde die Extraktion ohne Membran in Schüttelversuchen durchgeführt. Diese wurden mit verschiedenen Acrylsäurekonzentrationen in Wasser und Toluol als Extraktionsmittel im Verhältnis 2:1 (Wasser: Toluol) durchgeführt. Bei gleichem Energieeintrag wurden die zweiphasigen Lösungen aus Acrylsäure, Wasser und Toluol (stabilisiert mit 4-HT) dispergiert und anschließend das Absetzverhalten beobachtet.

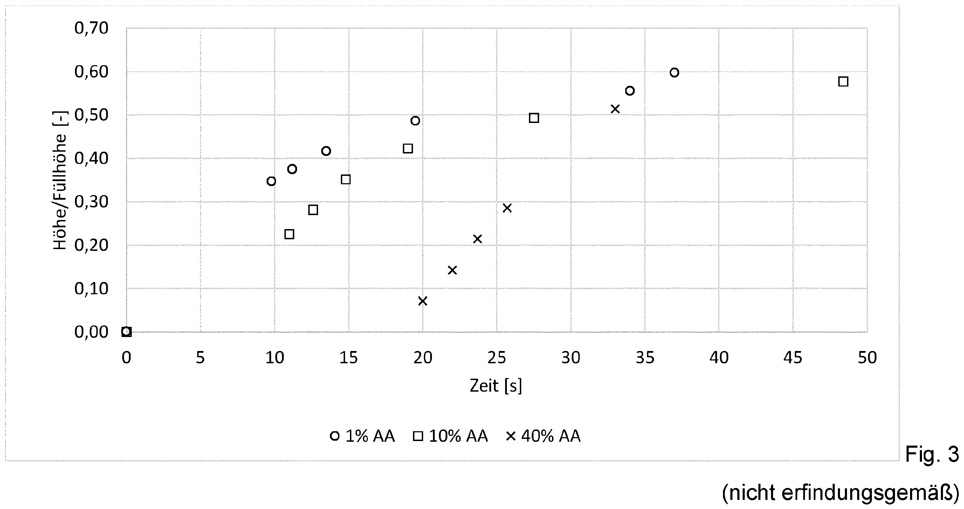

In dem in Figur 3 dargestellten Diagramm ist die Höhe, an der sich die Phasengrenze befand, gegen die Zeit nach Dispergierung aufgetragen. Die Höhe ist auf die Gesamtfüllhöhe bezogen und daher dimensionslos. Die Zeit ist in Sekunden angegeben. Die runden Punkte (∘) beziehen sich auf die Messpunkte mit 1%-iger Acrylsäure (AA), die quadratischen Punkte (_{□}) beziehen sich auf die Messpunkte mit 10%-iger Acrylsäure (AA), die gekreuzten Punkte (x) beziehen sich auf die Messpunkte mit 40%-iger Acrylsäure (AA). Die jeweiligen Punkte bilden eine Sedimentationslinie (nicht dargestellt).

Die Sedimentationsgeschwindigkeit vervierfacht sich bei Reduktion der eingesetzten Acrylsäurekonzentration in Wasser von 40% (0,6 mm/s) auf 1% (2,4 mm/s). Die dazugehörigen Daten sind in Tabelle 2 dargestellt.

Normalerweise muss in einem System aus Wasser und Toluol viel Energie aufgewendet werden, um Tropfen zu erzeugen, weil die Grenzflächenspannung so hoch ist. Deswegen wurden im Stand der Technik auch KARR-Kolonnen für diese Extraktionsaufgabe eingesetzt - mit den eingangs beschriebenen Nachteilen. Durch die vorliegenden Schüttelversuche wurde gezeigt, dass durch Anwensenheit von "viel" Acrylsäure die Grenzflächenspannung offensichtlich herabgesetzt wird und Tropfen mit kleinerem Energieeintrag erzeugt werden können. Dementsprechend ist der nötige Energieeintrag für die Dispergierung bei der 1%igen Acrylsäure in Wasser mit Toluol deutlich höher als im Fall der 40%igen Acrylsäure in Wasser mit Toluol.

Daher kann eine Kombination aus Kolonne und Membrankontaktor, je nach nötigem Energieeintrag, sinnvoll sein. Das Optimum ergibt sich aus einer Wirtschaftlichkeitsrechnung.

**Tabelle 2: Sedimentationsgeschwindigkeit in Abhängigkeit von der eingesetzten Konzentration der Acrylsäure in wässriger Phase in einem Phasenverhältnis von wässriger Phase zu Toluol.**

| | Dichtedifferenz wässrige Phase - organische Phase | Sedimentgeschwindigkeit [mm/s] |
|---|---|---|
| 1 % Acrylsäure | 133 | 2,4 |
| 10 % Acrylsäure | 129 | 1,6 |
| 40 % Acrylsäure | 117 | 0,6 |

### Bezugszeichen

- 0: Membrankontaktor
- 1: erster Behälter (Lumen - Wasser/Acrylsäure)
- 2: zweiter Behälter (Shell - Toluol)
- 3: erste Pumpe
- 4: zweite Pumpe
- 5: erster Thermostat mit Wärmetauscher
- 6: zweiter Thermostat mit Wärmetauscher
- 7: dritter Thermostat

## Patentansprüche

1. Verfahren zur Extraktion von Acrylsäure aus wässrigen Strömen, **dadurch gekennzeichnet, dass** ein wässriger Strom enthaltend Acrylsäure mit einer Membran in Kontakt gebracht wird, deren von dem wässrigen Strom abgewandten Seite mit einem organischen Lösemittel beaufschlagt wird.

2. Verfahren nach Anspruch 1, **dadurch gekennzeichnet, dass** die Membran poröses Polytetrafluorethylen enthält oder dass die Membran aus porösem Polytetrafluorethylen besteht.

3. Verfahren nach Anspruch 1 oder 2, **dadurch gekennzeichnet, dass** die Membran in Gestalt eines Hohlfaser-Moduls vorliegt, das im Gegenstrom betrieben wird.

4. Verfahren nach Anspruch 1, 2 oder 3, **dadurch gekennzeichnet, dass** es bei einer Temperatur von 20°C bis 30°C oder von 20°C bis 50°C durchgeführt wird.

5. Verfahren nach einem der Ansprüche 1 bis 4, **dadurch gekennzeichnet, dass** das organische Lösemittel mindestens eine Substanz enthält, die ausgewählt ist aus der Gruppe bestehend aus Toluol, n-Heptan, Essigsäureisobutylester, Essigsäure-n-propylester, Essigsäureisopropylester, 2-Pentanon, Methylisobutylketon.

6. Verfahren nach Anspruch 5, **dadurch gekennzeichnet, dass** Toluol als organisches Lösemittel eingesetzt wird.

7. Verfahren nach einem der vorhergehenden Ansprüche, **dadurch gekennzeichnet, dass** die Membran unbewegt ist.

8. Verfahren nach einem der vorhergehenden Ansprüche, **dadurch gekennzeichnet, dass** das organische Lösemittel im Kreislauf zirkuliert wird, wobei der Kreislauf die Beaufschlagung der Membran mit dem organischen Lösemittel und eine destillative Abtrennung des organischen Lösemittels aus der Acrylsäure umfasst.

9. Verfahren nach einem der vorhergehenden Ansprüche, **dadurch gekennzeichnet, dass** das organische Lösemittel shellseitig gefahren wird, währenddessen der wässrigen Strom lumenseitig gefahren wird.

10. Verfahren nach einem der Ansprüche 1 bis 9, durchgeführt mit einer hydrophoben Membran, **gekennzeichnet durch** ein Druckgefälle, welches zwischen der einen under der anderen Seite der Membran herrscht, wobei der Druck auf der dem wässrigen Strom abgewandten Seite zwischen 1000 Pa und 10000 Pa niedriger liegt als der Druck auf der dem wässrigen Strom zugewandten Seite.

11. Verfahren nach einem der Ansprüche 1 bis 9, durchgeführt mit einer hydrophilierten Membran, **gekennzeichnet durch** ein Druckgefälle, welches zwischen der einen under der anderen Seite der Membran herrscht, wobei der Druck auf der dem wässrigen Strom abgewandten Seite zwischen 1000 Pa und 10000 Pa höher liegt als der Druck auf der dem wässrigen Strom zugewandten Seite.

12. Verfahren nach einem der vorhergehenden Ansprüche, **dadurch gekennzeichnet, dass** der wässrige Strom einen ersten Inhibitor enthält und dass das organische Lösemittel einen zweiten Inhibitor enthält, wobei der erste Inhibitor und der zweite Inhibitor gleich oder unterschiedlich sind.

13. Verfahren zur Extraktion von Acrylsäure aus organischen Strömen, **dadurch gekennzeichnet, dass** ein organischer Strom enthaltend Acrylsäure mit einer Membran in Kontakt gebracht wird, deren von dem organischen Strom abgewandten Seite mit einem wässrigen Lösemittel beaufschlagt wird.

14. Verfahren zur Herstellung von Acrylsäure, umfassend einen ersten Reaktionsschritt, bei dem Propen mit Sauerstoff zu Acrolein umgesetzt wird, einen zweiten Reaktionsschritt, bei dem Acrolein mit Sauerstoff zu Acrylsäure umgesetzt wird, und einen Absorbtionsschritt, bei dem Acrylsäure in einem wässrigen Strom absorbiert wird, **gekennzeichnet durch** einen Extraktionsschritt, bei dem Acrylsäure zumindest teilweise mit einem Verfahren nach einem der Ansprüche 1 bis 12aus dem wässrigen Strom extrahiert wird.

15. Verfahren zur Herstellung von Acrolein, umfassend einen Reaktionsschritt, bei dem Propen mit Sauerstoff zu Acrolein und Acrylsäure umgesetzt wird und weiter umfassend einen Absorbtionsschritt, bei dem Acrylsäure in einem wässrigen Strom absorbiert wird, **gekennzeichnet durch** einen Extraktionsschritt, bei dem Acrylsäure zumindest teilweise mit einem Verfahren nach einem der Ansprüche 1 bis 12 aus dem wässrigen Strom extrahiert wird.
